Europäisches Patentamt

European Patent Office

Office européen des brevets

(11) Veröffentlichungsnummer: **0 322 538**
**A1**

## (12) EUROPÄISCHE PATENTANMELDUNG

(21) Anmeldenummer: 88117968.3

(22) Anmeldetag: 28.10.88

(51) Int. Cl.⁴: **D06B 3/04 , A41B 13/02**

(30) Priorität: 31.12.87 DE 3744587

(43) Veröffentlichungstag der Anmeldung:
05.07.89 Patentblatt 89/27

(84) Benannte Vertragsstaaten:
BE CH ES FR GB IT LI NL SE

(71) Anmelder: **MACON KLEBETECHNIK GMBH**
**Max-Planck-Strasse 15**
**D-4006 Erkrath 1(DE)**

(72) Erfinder: **Rothen, Josef**
**Höher Heide 60**
**D-5650 Solingen 11(DE)**

(74) Vertreter: **Palgen, Peter, Dipl.-Phys. Dr.**
**Mulvanystrasse 2**
**D-4000 Düsseldorf(DE)**

(54) Verfahren zum Auftragen von Leim auf endlose Fäden und entsprechende Vorrichtung.

(57) Um einen Faden der Länge nach gleichmäßig mit einem Leimauftrag zu versehen, wird der Faden der Länge nach durch die Längsbohrung (3) einer Düsennadel (4) und anschließend durch die Düsenbohrung (16) einer Düsenplatte (15) hindurchgeführt. Die Düsennadel (4) dient gleichzeitig als Ventilelement und erlaubt das Umspritzen des Fadens mit Leim, der unter Druck einer das vordere Ende der Düsennadel (4) umgebenden Kammer (19) zugeführt wird. Der Auftragskopf (10) dient insbesondere zum Beleimen der gummielastischen Fäden, die am Rand der Beinausschnitte von Baby-Windelhöschen angebracht werden.

Fig.1

## Verfahren zum Auftragen von Leim auf endlose Fäden und entsprechende Vorrichtung

Die Erfindung bezieht sich auf ein Verfahren nach dem Oberbegriff des Anspruchs 1 und eine entsprechende Vorrichtung.

Ausgangspunkt der Erfindung sind Probleme gewesen, die bei der Herstellung von Baby-Windelhöschen auftreten. Diese Windelhöschen bestehen aus einem im wesentlichen rechteckigen Flächengebilde, welches auf der Außenseite eine sehr biegeschlaffe undurchlässige Folie und auf der Innenseite ein saugfähiges Polster aufweist. An den beiden Längsseiten sind längliche Beinausschnitte vorgesehen. Die über die Beinausschnitte in Längsrichtung überstehenden Bereiche des Windelhöschens werden vorn und hinten um den Körper herumgelegt und an den Seiten mit einem an dem Windelhöschen vorhandenen Zuschnitt einer Klebefolie verklebt. Die Beinausschnitte sollen sich dem Bein sanft jedoch dicht anschmiegen, so daß an dieser Stelle keine Flüssigkeit austreten kann. Zur Erzielung dieser Schmiegsamkeit ist das Flächengebilde im Bereich der Beinausschnitte gekreppt und somit in einem gewissen Grade elastisch dehnbar. Die Kreppung wird durch einen oder mehreren in Längsrichtung nebeneinander innerhalb des Randes des Beinausschnittes angeordnete gummielastische Fäden erzeugt, die in gedehntem Zustand in dem Bereich der Beinausschnitte auf ihrer ganzen Länge mit dem Flächengebilde verklebt werden. In den in Längsrichtung über die Beinausschnitte überstehenden Bereichen soll keine Verklebung stattfinden, damit diese Bereiche nicht verkreppt werden und flachgefaltet werden können. Die gummielastischen Fäden können aus Gummimaterial oder aus Textilmaterial bestehen, welches durch eine Texturierung eine Gummielastizität in Längsrichtung erhalten hat, z.B. aus "Lycra" ( = eingetragenes Warenzeichen der Firma E.I. Du Pont de Nemours and Company).

Es sind zwei Verfahren bekannt,um die längenweise Verklebung der gummielastischen Fäden mit dem Flächengebilde des Windelhöschens zustande zu bringen. Bei dem ersten Verfahren wird auf dem Flächengebilde eine Leimspur erzeugt, in die hinhein die gummielastischen Fäden unter Vorspannung gedrückt werden. Dieses Verfahren ist verbesserungsfähig, weil die Haftung des Leims an den gummielastischen Fäden unbefriedigend ist und durch die Leimspur eine Verhärtung des Flächengebildes in einer Zone stattfindet, wo sie am wenigsten erwünscht ist, nämlich im Bereich des Randes der Beinausschnitte.

Bei einem anderen Verfahren wird der Leim auf die Fäden aufgetragen, indem diese durch einen Leimvorrat hindurchgezogen werden. Hierbei ist die Haftfähigkeit der Fäden auf dem Flächengebilde ebenfalls unbefriedigend, weil die Fäden den Leim nicht bereitwillig annehmen und häufig nur einzelne in Abständen vorhandene Leimtropfen an dem Faden hängen bleiben.

Ausgehend von diesen speziellen Problemen liegt der Erfindung die Aufgabe zugrunde, ein Verfahren der dem Oberbegriff des Anspruchs 1 entsprechenden Art sowie eine entsprechende Vorrichtung anzugeben, mit denen Leim auf Fäden so aufgetragen werden kann, daß eine sichere Verklebung des Fadens mit einem Flächengebilde möglich ist.

Diese Aufgabe wird in ihrem verfahrensmäßigen Aspekt durch die in Anspruch 1 wiedergegebene Erfindung gelöst.

Überraschenderweise hat sich gezeigt, daß, wenn der Faden mit dem Leim umspritzt wird, d.h. der Leim unter einem gewissen allseitigen Druck mit dem Faden zusammengebracht wird, eine weitgehend gleichmäßige Beschichtung der Fadenoberfläche mit Leim erzielbar ist, die eine gute Verklebung des Fadens auf seiner Länge mit dem Flächengebilde ermöglicht.

Die Erfindung ist auch in dem in Anspruch 2 wiedergegebenen Verfahren verwirklicht, welches speziell auf die Herstellung von Baby-Windelhöschen gerichtet ist.

Besonders vorteilhaft ist hierbei, daß der Leim leicht auf den schon gedehnten Faden aufgebracht werden und durch Unterbrechung des Umspritzens die Länge der beleimten Abschnitte genau bestimmt werden kann, so daß nur auf die Längenabschnitte des Fadens, die neben die Beinauschnitte zu liegen kommen, Leim aufgetragen wird.

In ihrem vorrichtungsmäßigen Aspekt ist die Erfindung in Anspruch 3 wiedergegeben.

Der zugrundeliegende Gedanke kann insbesondere in der in Anspruch 4 beschriebenen Weise konkret verwirklicht werden.

Der Faden läuft nacheinander durch die Bohrung der Düsennadel und die Düsenbohrung und wird bei vom Ventilsitz zurückgezogener Düsennadel durch den aus der umgebenden Kammer herandrängenden Leim beschichtet. Der Faden tritt mit dieser Beschichtung duch die Düsenbohrung hin durch. Sobald die Düsennadel gegen den Ventilsitz in dem Düsenelement vorgeschoben wird, bricht die Leimzufuhr ab und endet die Beschichtung, so daß der Faden allenfalls noch geringfügige, in der Düsenbohrung verbliebene Leimmengen mitnimmt und sich ein exakter Abriß der Beleimung ergibt.

Die Vorrichtung ist gleichermaßen für gummielastische Fäden geeignet, die in gedehnter Form mit Leim beschichtet werden und für Anwendungsfälle wie Baby-Windelhöschen vorgesehen sind, als auch für normale Fäden, die keine wesentliche Dehnung in Längsrichtung zeigen und z.B. zu Verstärkungszwecken auf Papiersäcke und dergleichen aufgebracht werden.

Eine zweckmäßige Ausführungsform des Antriebs ist ein druckmittelbetätigter Kolben (Anspruch 5) wobei aber auch andere Antriebsarten, z.B. ein elektromagnetischer Antrieb, nicht ausgeschlossen sind.

Die Bohrung der Düsennadel wird an dem vorderen Ende ihren engsten Querschnitt aufweisen, durch den der Faden gerade noch hindurchpaßt, ohne hängenzubleiben. Die Düsenbohrung weist zweckmäßig einen etwas größeren Durchmesser auf (Anspruch 6), damit die Leimschicht auf dem Faden Platz hat und der im Bereich des Ventilsitzes aufgetragene Leim nicht sogleich wieder abgestreift wird. Der Durchmesserunterschied dosiert bei einer vorgegebenen Geschwindigkeit des Fadens die aufgetragene Leimmenge.

Durch die Ausgestaltung des Düsenelements als Düsenplatte gemäß Anspruch 7, die in der Praxis eine Dicke von beispielsweise 3 mm aufweist, ist die Länge des Düsenkanals gering. Durch den Eingriff der Düsennadel in die Ansenkung der Düsenplatte erfolgt die Unterbrechung des Leims im Bereich des Düsenkanals, so daß insgesamt nach dem Schließen des durch die Düsennadel gebildeten Leimventils nur noch eine minimale Leimmenge in dem vorderen Teil der Düsenbohrung verbleibt und sogleich von dem Faden mitgenommen wird.

Anspruch 8 gibt eine zweckmäßige Ausgestaltung wieder, durch die sichergestellt ist, daß bei einem Ausfall des Druckmitteldrucks die Düsennadel in die Geschlossenstellung bewegt wird und kein Leim aus der Düsenbohrung austreten kann.

Maßgeblich für die Erfindung ist, daß der Faden in einer Düse unter einem gewissen Druck von allen Seiten mit dem Leim umspritzt wird, so daß sich eine geschlossene Leimhat zwangsweise bildet, die dann auch beim Austritt des Fadens aus der Düse auf dem Faden erhalten bleibt.

Die Durchführung dieses Gedankens ist nicht an die spezielle Ausführung der Düse gebunden, wie sie vorstehend beschrieben ist. Auch andere Ausführungsformen von Düsen sind zur Realisierung der Erfindung geeignet.

In Anspruch 9 ist ein Ausführungsbeispiel wiedergegeben, bei welchem der Leim nicht vorwiegend axial, sondern radial zugeführt wird.

Wenn der Leimauftrag an einer bestimmten Stelle des Fadens beendet werden soll und die Ventilanordnung daher geschlossen wird, kann es in der Zuführleitung zu Druckstößen und bis zum Wiederöffnen der Ventilanordnung zu einer Druckerhöhung kommen, die dazu führt, daß im ersten Moment der erneuten Öffnung eine größere Leimmenge aufgetragen wird, bis sich in der Strömung der Zuführleitung wieder stationäre Verhältnisse eingestellt haben.

Um einen derartigen ungleichmäßigen Leimauftrag zu vermeiden, kann nach einer weiteren Ausgestaltung der Erfindung gemäß Anspruch 10 ein Rückführkanal mit einer Ventilanordnung vorgesehen sein, mittels deren der geförderte Leim beim Schließen des Leimaustritts zur Pumpe zurückführbar ist. Die Pumpe arbeitet also stetig und ist auf diese Weise vom Schließen des Leimaustritts überhaupt nicht betroffen. Der Druck in der Zuführleitung bleibt stets auf gleicher Höhe, ob nun der Leim am Leimaustritt auf dem Faden übertragen oder zurückgeführt wird. Auf diese Weise wird der Leimauftrag pro Einheit der Fadenlänge vergleichmäßigt.

In der Zeichnung sind Ausführungsbeispiele der Erfindung dargestellt.

Fig. 1 ist ein Längsschnitt durch den die Vorrichtung bildenden Auftragskopf;

Fig. 2 ist eine vergrößerte Wiedergabe des in Fig. 1 strichpunktiert umrahmten Bereiches;

Fig. 3 zeigt als Anwendungsbeispiel ein flachgelegtes Baby-Windelhöschen in der Phase der Anbringung der gummielastischen Fäden;

Fig. 4 zeigt das Windelhöschen nach Fig. 3 nach dem Abtrennen;

Fig. 5 zeigt eine abgewandelte Ausführungsform der Düse.

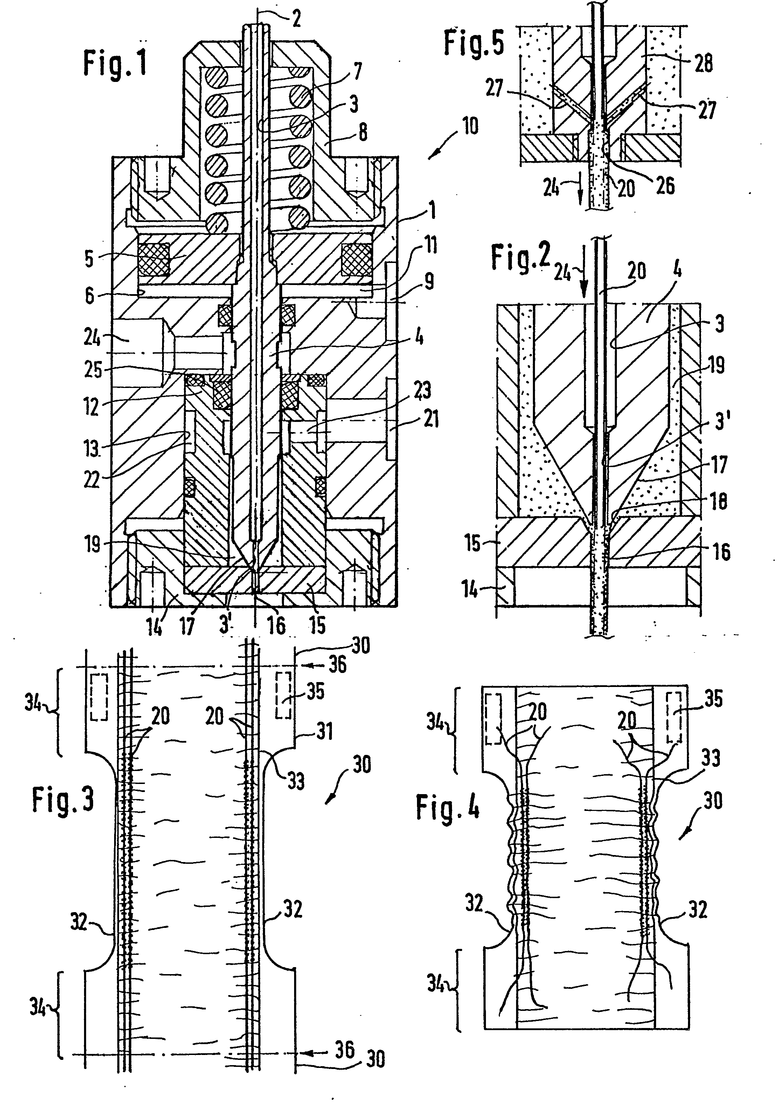

Der als Ganzes mit 10 bezeichnete Auftragskopf umfaßt ein blockartiges Gehäuse 1 mit mehreren zu einer Achse 2 koaxialen in Längsrichtung aufeinanderfolgenden Ausnehmungen. In den Ausnehmungen ist eine mit einer durchgehenden Längsbohrung 3 versehene Düsennadel 4 angeordnet, die in ihrer Längsrichtung verschiebbar ist. An ihrem "vorderen", in Fig. 1 untengelegenen Ende 17 ist die Außenseite der Düsennadel mit einem Öffnungswinkel von 60° konisch ausgebildet. Im Bereich des Konus besitzt die Düsennadel 4 einen Bohrungsteil 3' verringerten Durchmessers, durch den ein zu beleimender Faden 20 gerade hindurchpaßt, ohne daß an ihm stark gezogen werden muß. Der rückwärtige Teil der Bohrung 3 hat einen etwas größeren Durchmesser, so daß sich der Faden 20 frei hindurchbewegen kann, wie aus Fig. 2 ersichtlich ist.

Im mittleren Abschnitt ist auf die Düsennadel 4 ein scheibenförmiger Kolben 5 aufgeschraubt, der in einer zylindrischen Kammer 6 des Gehäuses in Achsrichtung der Düsennadel 4 beweglich ist. Gegen den Kolben 5 wirkt von oben eine Schraubendruckfeder 7, die den oberen Teil der Düsennadel 4 umgibt und in einem Deckel 8 untergebracht ist, der in das obere Ende des Gehäuses 1 eingeschraubt ist. Normalerweise wird also die Düsennadel 4 nach unten gedrückt. Über einen Einlaß 9 kann der unter dem Kolben 5 befindliche Teil 11 der zylindrischen Kammer 6 mit einem fluiden Druckmedium, z.B. Druckluft gefüllt werden, wodurch der Kolben 5 gegen die Kraft der Feder 7 nach oben bewegt wird und dabei die Düsennadel 4 mitnimmt.

Im unteren Bereich ist die Düsennadel 4 in einem zylindrischen Einsatz 12 geführt, der in einer zylindrischen Ausnehmung 13 des Gehäuses abgedichtet angeordnet und durch einen in das untere Ende des Gehäuses eingeschraubten Deckel 14 darin festgehalten ist. Zwischen dem Deckel 14 und der Stirnseite des zylindrischen Einsatzes 12 ist eine Düsenplatte 15 eingespannt, die eine Düsenbohrung 16 aufweist, die zu dem vorderen Teil 3' der Bohrung 3 der Düsennadel 4 koaxial ist und einen geringfügig größeren Durchmesser aufweist als der Teil 3'.

Die Düsennadel 4 kommt unter der Wirkung der Feder 7 mit ihrem konischen vorderen Ende 17 in einer entsprechend konischen Ansenkung 18 des der Düsennadel 4 zugewandten Endes der Düsenbohrung 16 zur Anlage.

Um das vordere Ende der Düsennadel 4 herum ist eine zylindrische Kammer 19 etwas größeren Durchmessers gebildet, die über einen Zuführkanal 21 für den Leim, einen am Außenumfang des zylindrischen Einsatzes 12 gebildeten Ringkanal 22 und mehrere von diesem ausgehende, über den Umfang verteilte Radialkanäle 23 mit Leim beschickbar ist. Der Leim gelangt bis in die Nähe der Spitze des vorderen Endes 17 der Hohlnadel 4. Dieses vordere Ende 17 wirkt mit dem einen Ventilsitz bildenden Ansenkung 18 ventilmäßig zusammen und sperrt, wenn in dem unteren Teil 11 der Kammer 6 kein Druck herrscht und die Ventilnadel 4 durch die Feder 7 in Anlage an der Düsenplatte 15 gehalten wird, den Übertritt von Leim aus der Kammer 19 in die Düsenbohrung 16 ab (Fig. 1). Wenn aber durch Einführung von Druckluft in den Teil 11 der Kammer 6 die Düsennadel 4 von der Ansenkung 18 zurückgezogen ist, tritt in der Fig. 2 angedeutete Weise Leim durch den konischen Spalt zwischen dem vorderen Ende 17 und der Ansenkung 18 hindurch in die Düsenbohrung 16 über und wird von dem im Sinne des Pfeiles 24 sich bewegenden Faden 20 mitgenommen, wobei der Leim eine den Faden 20 geschlossen umgebende und in sich zusammenhängende Schicht bildet. Damit die Leimschicht auf dem Umfang des Fadens 20 Platz hat, ist der Durchmesser der Düsenbohrung 16 etwas größer als der Durchmesser des Fadens 20 bzw. des Teils 3' der Bohrung 3.

Wenn der Druck unter dem Kolben 5 weggenommen wird, setzt sich das vordere Ende 17 wieder auf die Ausnehmung 18 und versperrt den Durchgang für den Leim. Der weiterlaufende Faden 20 erhält dann keinen Leim mehr, wobei sich ein sehr exakter Abriß der Beschichtung einstellt.

Damit sich in dem Zuführkanal 21 bei dem Schließen des Leimaustritts 17,18 kein Druckstoß ausbildet und der Druck bei geschlossenem Leimaustritt 17,18 auch nicht ansteigt, sind ein nicht dargestellter Rückführkanal und eine Ventilanordnung vorgesehen, mittels deren, wenn der Leimaustritt 17,18 geschlossen wird, automatisch eine Umsteuerung des durch den Zuführkanal 21 herangeführten Leims in den Rückführkanal und zur Pumpe stattfindet. Die Pumpe fördert also dauernd mit gleichbleibendem Druck, ob nun der Leimaustritt 17,18 geöffnet oder geschlossen ist. Auf diese Weise wird vermieden, daß bei erneutem Öffnen des Leimaustritts 17,18 nach einem geschlossenen Zustand im ersten Moment ein erhöhter Druck ansteht, der zu einem verstärkten Leimauftrag Anlaß ist, worauf sich der Leimauftrag allmählich erst wieder normalisiert. Diese Ungleichmäßigkeit wird durch den dauernd geschlossenen Leimkreislauf vermieden.

In Fig. 5 ist eine alternative Ausführungsform der Düse dargestellt. Der Faden 2o tritt durch eine feststehende der Düsennadel 4 entsprechende Nadel 28 hindurch, an deren vorderem Ende die Düse 26 gebildet ist, die durch über den Umfang verteilte von außen in die Düse 26 mündende Zuführkanäle 27 mit Leim versorgt wird, der dort auf den im Sinne des Pfeiles 24 vorlaufenden Faden 2o übertragen wird. Das Zurücksteigen in den einen größeren Durchmesser aufweisenden Teil des Kanals der Nadel 28 wird durch die Vorlaufbewegung des Fadens 2o unterbunden.

Der oberhalb des Einsatzes 12 einmündende Auslaß 24 dient zum Abziehen bei den Hubbewegungen der Düsennadel 4 etwa über die Dichtung 25 nach oben übergetretener geringer Leimmengen.

In Fig. 3 ist ein Anwendungsbeispiel in Gestalt eines Baby-Windelhöschens dargestellt. Das Baby-Windelhöschen 3o umfaßt einen im wesentlichen rechteckigen Zuschnitt 31 aus einer undurchlässigen Folie, der an den beiden Längsseiten einander gegenüberliegende Beinausschnitte 32 aufweist. An den kürzeren Seiten hängen aufeinanderfolgende Baby-Windelhöschen 3o noch aneinander und bilden eine fortlaufende Bahn. Auf die spätere Innenseite ist ein sich über die Länge des Baby-Windelhöschens 30 erstreckendes, bis nahe an den Rand der Beinausschnitte 32 reichendes stark saugfähiges und auch unter Druck flüssigkeitshaltendes Polster 33 aufgebracht.

Bei angelegtem Baby-Windelhöschen 30 befinden sich die über die Beinausschnitte in Längsrichtung überstehenden Bereiche 34 vor bzw. hinter dem Körper und werden an den Seiten durch Klebefolienzuschnitte 35 miteinander verbunden. Die Beinausschnitte 32 umgeben dann den Oberschenkel des Babys ringsherum und sollen möglichst dicht anliegen. Zu diesem Zweck werden dicht innerhalb des Randes des Beinauschnittes 32 zwei gummielastische Fäden 20 in gedehntem Zustand aufgeklebt, die in einem Auftragskopf 10 nach den Fig. 1 und 2 mit Leim beschichtet worden sind. Statt zwei können natürlich auch drei oder vier gummielastische Fäden 20 pro Seite vorgesehen sein. Für jeden Faden 20 ist ein Auftragskopf 10 vorgesehen. Die verschiedenen Auftragsköpfe 10 sind in geeigneter Weise gestaffelt angeordnet, damit die Fäden gerade im Sinn der Achse 2 verlaufen können und dennoch sehr dicht nebeneinander auf das das Baby-Windelhöschen 30 bildende Flächengebilde aufgelegt werden können, z.B. während das Band der Baby-Windelhöschen 30 eine Trommel umrundet.

Die Fäden 20 sind nur im Bereich der Längenerstreckung der Beinausschnitte 32 mit Leim versehen, in den überstehenden Bereichen 34 jedoch leimfrei. Der Sinn dieser Maßnahme ergibt sich aus Fig. 4. Nach dem Aufkleben der Fäden 20 werden die einzelnen Baby-Windelhöschen 30 entlang der Schnittlinien 36 abgetrennt. Da die Fäden 20 in gedehntem Zustand mit dem Baby-Windelhöschen 30 verklebt worden sind, ziehen sie sich im Bereich der Beinausschnitte 32 zusammen, sobald die Abtrennung entlang der Linien 36 erfolgt ist, und bewirken im Bereich der Beinausschnitte 32 eine in Fig. 4 angedeutete Kreppung, d.h. eine Verkürzung des Materials, die diesem umgekehrt wieder eine Dehnfähigkeit verleiht und für den dichten Schluß im Bereich der Beinausschnitte 32 verantwortlich ist.

Die Kreppung soll sich aber auf den Bereich der Beinausschnitte 32 beschränken. Die in Achsrichtung überstehenden Bereiche 34 sollen eben verbleiben. Dies ist sowohl im Hinblick auf das möglichst flache Zusammenlegen der fertigen Baby-Windelhöschen 30 als auch im Hinblick auf das Anlegen bei der Benutzung notwendig. Die in den Bereichen 34 gelegenen Abschnitte der gedehnten Fäden 20 waren nicht beleimt und schnappen daher in der in Fig. 4 angedeuteten Weise frei zurück, ohne auf das Material in den Abschnitten 34 einen Zug oder eine Verformung auszuüben.

Als "Leim" soll in erster Linie Heißschmelzkleber verstanden sein, obwohl andere Leimarten ebenso mit Vorteil verarbeitet werden können.

**Ansprüche**

1. Verfahren zum Auftragen von Leim auf einen endlosen Faden, der mindestens auf Abschnitten seiner Länge mit einem Flächengebilde verklebt werden soll, dadurch gekennzeichnet, daß der Faden durch eine Düse hindurchgeführt und darin mit dem Leim umspritzt wird.

2. Verfahren zur Herstellung von Baby-Windelhöschen aus einem im wesentlichen rechteckigen Flächengebilde, welches an den längeren Seiten Beinausschnitte und dicht innerhalb der Beinausschnitte in Längsrichtung des Flächengebildes verlaufende gummielastische Fäden aufweist, die in gedehntem Zustand im Bereich der Beinausschnitte mit dem Flächengebilde auf der ganzen Länge verklebt, in den über die Beinausschnitte in Längsrichtung überstehenden Bereichen jedoch nicht verklebt sind, dadurch gekennzeichnet, daß die gummielastischen Fäden in gedehntem Zustand durch eine Düse hindurchgeführt und darin auf der Länge der Beinausschnitte entsprechenden Längenabschnitten mit Leim umspritzt werden.

3. Vorrichtung zum Auftragen von Leim auf vorlaufende endlose Fäden, dadurch gekennzeichnet, daß sie eine Düse (15, 16) umfaßt, durch die der Faden (20) hindurch führbar und in der er mit unter Druck zugeführtem Leim umspritzbar ist.

4. Vorrichtung nach Anspruch 3, gekennzeichnet durch folgende Merkmale:

a) ein Gehäuse (1);

b) eine in dem Gehäuse (1) parallel zu ihrer Längsachse (2) verlagerbare, der Länge nach durchbohrte Düsennadel (4), durch die der Faden (20) hindurchführbar und die am vorderen Ende (17) als Ventilelement ausgebildet ist;

c) ein im Gehäuse (1) angeordnetes Düsenelement (15) mit einer zur Bohrung (3, 3') der Düsennadel (4) koaxialen Düsenbohrung (16), durch die der Faden (20) ebenfalls hindurchführbar ist, und einem zum ventilmäßigen Zusammenwirken mit dem vorderen Ende (17) der Düsennadel (4) ausgebildeten Ventilsitz (18);

d) eine um das vordere Ende (17) der Düsennadel (4) herum in dem Gehäuse (1) vorgesehene Kammer (19), die einen Anschluß (21) für den Leim aufweist;

e) einen Antrieb, mittels dessen die Düsennadel (4) gesteuert zum Unterbrechen des Leimaustritts aus der Kammer (19) in die Düsenbohrung (16) bis zur Anlage am Ventilsitz (18) gegen das Düsenelement (15) vorschiebbar und zur Freigabe des Leimaustritts zurückziehbar ist.

5. Vorrichtung nach Anspruch 4, dadurch gekennzeichnet, daß der Antrieb einen mit der Düsennadel (4) verbundenen Kolben (5) umfaßt, der in einer zylindrischen Kammer (6) des Gehäuses (1) in Achsrichtung der Düsennadel (4) unter dem Druck eines fluiden Mediums verschiebbar ist.

6. Vorrichtung nach einem der Ansprüche 3 bis 5, dadurch gekennzeichnet, daß die Düsenbohrung (16) einen um einen den Leimauftrag dosierenden Betrag größeren Durchmesser aufweist als die Bohrung (3') am vorderen Ende der Düsennadel (4).

7. Vorrichtung nach einem der Ansprüche 4 bis 6, dadurch gekennzeichnet, daß das Düsenelement (15) als Düsenplatte ausgebildet ist und an dem der Düsennadel (4) zugewandten Ende der Düsenbohrung (16) eine als Ventilsitz dienende Ansenkung (18) aufweist, in die die Düsennadel (4) mit ihrem außen entsprechend konischen vorderen Ende (17) eingreift.

8. Vorrichtung nach einem der Ansprüche 4 bis 7, dadurch gekennzeichnet, daß eine Feder (7) vorgesehen ist, die die Düsennadel (4) normalerweise in Anlage am Ventilsitz (18) hält und gegen deren Wirkung das fluide Medium die Düsennadel (4) vom Ventilsitz (18) abhebt.

9. Vorrichtung nach Anspruch 3, dadurch gekennzeichnet, daß eine Düse vorgesehen ist, in deren Düsenbohrung über den Umfang verteilte Zuführkanäle für den Leim von außen einmünden.

10. Vorrichtung nach einem der Ansprüche 3 bis 9, dadurch gekennzeichnet, daß ihr ein Rückführkanal mit einer Ventilanordnung zugeordnet ist, mittels deren der durch den Zuführkanal (21) geförderte Leim beim Schließen des Leimaustritts (17,18) zur Pumpe zurückführbar ist.

Fig.1

Fig.5

Fig.2

Fig.3

Fig.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4 ) |
|---|---|---|---|
| X | US-A-2608950 (UNITED SHOE MACHINERY CORPORATION) <br> * das ganze Dokument * <br> --- | 1, 3 | D06B3/04 <br> A41B13/02 |
| A | FR-A-2274241 (RIEGEL TEXTILE CORPORATION) <br> --- | 2 | |
| A | FR-A-2240174 (JOHNSON & JOHNSON) <br> --- | 2 | |
| A | US-A-4316312 (ALLIED CORPORATION) <br> --- | | |
| A | FR-A-2226818 (RHONE-POULENC-TEXTILE) <br> ----- | | |

**RECHERCHIERTE SACHGEBIETE (Int. Cl.4 )**

D06B
A41B

Der vorliegende Recherchenbericht wurde für alle Patentansprüche erstellt

| Recherchenort | Abschlußdatum der Recherche | Prüfer |
|---|---|---|
| DEN HAAG | 15 DECEMBER 1988 | PETIT J.P. |